(19) Europäisches Patentamt / European Patent Office / Office européen des brevets

(11) **EP 4 551 108 B1**

(12) **EUROPEAN PATENT SPECIFICATION**

(45) Date of publication and mention
of the grant of the patent:
**29.04.2026   Bulletin 2026/18**

(21) Application number: **23745637.1**

(22) Date of filing: **06.07.2023**

(51) International Patent Classification (IPC):
*A61B 5/0536* (2021.01)    *A61B 5/08* (2006.01)
*A61B 5/085* (2006.01)    *A61B 5/087* (2006.01)
*A61B 5/00* (2006.01)    *A61M 16/00* (2006.01)

(52) Cooperative Patent Classification (CPC):
(C-Sets available)
**A61M 16/024; A61B 5/0536; A61B 5/086;
A61B 5/4836; A61B 5/6823; A61M 16/201;**
A61M 16/0833; A61M 16/0883; A61M 16/205;
A61M 16/208; A61M 2016/0015; A61M 2016/0027;
A61M 2016/003; A61M 2205/15; A61M 2205/3317;
(Cont.)

(86) International application number:
**PCT/IB2023/056994**

(87) International publication number:
**WO 2024/009255 (11.01.2024 Gazette 2024/02)**

(54) **A SYSTEM FOR DETECTING ASYNCHRONOUS RESPIRATORY EVENTS AND ASSOCIATED METHODS AND COMPONENTS**

SYSTEM ZUR ERKENNUNG ASYNCHRONER ATMUNGSEREIGNISSE UND ZUGEHÖRIGE VERFAHREN UND KOMPONENTEN

SYSTÈME DE DÉTECTION D'ÉVÉNEMENTS RESPIRATOIRES ASYNCHRONES ET PROCÉDÉS ET COMPOSANTS ASSOCIÉS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:   **06.07.2022   US 202263367807 P**

(43) Date of publication of application:
**14.05.2025   Bulletin 2025/20**

(73) Proprietor: **TIMPEL MEDICAL B.V.
5611 BJ Eindhoven (NL)**

(72) Inventor: **LEITE VIEIRA COSTA, Eduardo
CEP 04022-001 São Paulo-SP (BR)**

(74) Representative: **Studio Torta S.p.A.
Via Viotti, 9
10121 Torino (IT)**

(56) References cited:
WO-A1-2021/074747      US-A1- 2019 246 949
US-A1- 2021 016 035

**(Cont. next page)**

(52) Cooperative Patent Classification (CPC): (Cont.)
A61M 2205/3331; A61M 2205/3334;
A61M 2205/3553; A61M 2205/3584;
A61M 2205/3592; A61M 2205/505; A61M 2209/01;
A61M 2210/1039; A61M 2230/46; A61M 2230/65;
G16H 20/40; G16H 50/30

C-Sets
A61M 2230/46, A61M 2230/005;
A61M 2230/65, A61M 2230/005

**Description**

TECHNICAL FIELD

Embodiments of the present disclosure generally relate to medical support systems. In particular, embodiments of the present disclosure relate to systems for detecting asynchronous respiratory events and associated methods and components.

BACKGROUND

Mechanical ventilation is the treatment commonly used in hospitals to assist the breathing of a patient, such as during respiratory failure, anesthesia, and the treatment of other respiratory ailments, such as acute respiratory distress syndrome ("ARDS"), and others to maintain or restore a patient's breathing. Such control of human breathing involves a complex feedback mechanism between the nervous system - chemoreceptors (central and peripheral), mechanoreceptors - and vagal inputs of the lung, the lung itself, chest wall, and respiratory muscles. When mechanical ventilation is used, the synchrony between the ventilator and the patient's respiratory rhythm makes it possible to reduce the load of inspiratory muscles effectively and safely, as well as ensures the body oxygenation. Furthermore, asynchronous events between the mechanical ventilator and a patient's respiratory rhythm may reduce the efficiency of the mechanical ventilator and have the potential to cause injuries to the patient, such as by overinflating or underinflating the patients lungs.

An example of system for determining a respiratory effort of a patient is known from document WO2021/074747 A1.

There is therefore a substantial need for improved systems for detecting respiratory abnormalities in a patient.

DISCLOSURE

The aforementioned aim is reached by a system configured to detect asynchronous respiratory events and by a method of detecting an asynchronous respiratory event as claimed in the appended set of claims.

BRIEF DESCRIPTION OF THE SEVERAL VIEWS OF THE DRAWINGS

To easily identify the discussion of any particular element or act, the most significant digit or digits in a reference number refer to the figure number in which that element is first introduced.

FIG. 1 is a schematic diagram of a portion of an EIT system showing a plurality of electrodes positioned around a region of interest of a patient according to one or more embodiments of the disclosure;
FIG. 2 is a schematic diagram showing a cross-section of the thorax of the patient along the plane of the electrodes according to one or more embodiments of the disclosure;
FIG. 3 is a schematic block diagram of an EIT system according to an embodiment of the disclosure;
FIG. 4 shows a schematic view of a system for mechanically ventilating and monitoring a patient's lungs according to one or more embodiments of the disclosure;
FIG. 5 illustrates a flow chart representative of a process of evaluating respiratory cycles in accordance with embodiments of the disclosure;
FIGS. 6A and 6B illustrate flow waveforms and pressure waveforms in accordance with embodiments of the disclosure;
FIG. 7 illustrates a flow chart representative of a decision process in accordance with embodiments of the disclosure;
FIG. 8 illustrates a lung volume waveform of a respiratory cycle in accordance with embodiments of the disclosure;
FIG. 9 illustrates a lung volume waveform, a pressure waveform, and a flow waveform aligned in accordance with embodiments of the disclosure; and
FIGS. 10A and 10B illustrate EIT images of a patient's lungs in accordance with embodiments of the disclosure.

DESCRIPTION

The illustrations presented herein are not meant to be actual views of any particular system, method, or component thereof, but are merely idealized representations employed to describe illustrative embodiments. The drawings are not necessarily to scale.

As used herein, the term "substantially" in reference to a given parameter means and includes to a degree that one skilled in the art would understand that the given parameter, property, or condition is met with a small degree of variance, such as within acceptable manufacturing tolerances. For example, a parameter that is substantially met may be at least about 90% met, at least about 95% met, at least about 99% met, or even at least about 100% met.

As used herein, "about" in reference to a numerical value for a particular parameter is inclusive of the numerical value and a degree of variance from the numerical value that one of ordinary skill in the art would understand is within acceptable tolerances for the particular parameter. For example, "about" in reference to a numerical value may include additional numerical values within a range of from 90.0 percent to 110.0 percent of the numerical value, such as within a range of from 95.0 percent to 105.0 percent of the numerical value, within a range of from 97.5 percent to 102.5 percent of the numerical value, within a range of from 99.0 percent to 101.0 percent of the numerical value, within a range of from 99.5 percent to 100.5 percent of the numerical value, or within a range of from 99.9 percent to 100.1 percent of the numerical value.

As used herein, relational terms, such as "first," "second," "top," "bottom," "upper," "lower," "right," "left," etc., are generally used for clarity and convenience in understanding the disclosure and accompanying drawings and do not connote or depend on any specific preference, orientation, or order, except where the context clearly indicates otherwise.

As used herein, the term "and/or" means and includes any and all combinations of one or more of the associated listed items.

As used herein, the terms "vertical" and "horizontal" refer to the orientations as depicted in the figures.

Mechanical ventilation (MV) is the treatment commonly used in hospitals to maintain and/or assist patient breathing. When assisted mechanical ventilation is used, the synchrony between the ventilator and the patient's respiratory rhythm makes it possible to reduce the load of inspiratory muscles effectively and safely, as well as ensures the body oxygenation. Patient-ventilator asynchrony (PVA) may be defined as a mismatch between the patient's needs and the time, flow, tidal volume and/or pressure waveforms delivered according to the ventilator settings. PVAs may be subtle changes that can be viewed in the time, flow, tidal volume and/or pressure waveforms during monitoring of respiratory mechanics or even in monitoring the patient's clinical condition. However, their detection requires expert, real-time attention from a trained professional at the bedside. Identifying PVAs when a trained professional is not present may reduce the occurrence of PVA's by reducing the time before the PVA's are detected and adjustments are made to the mechanical ventilation system, which may reduce the detrimental effects of PVA's on the patient.

Embodiments of the disclosure may include an electrical impedance tomography (EIT) device configured to measure regional distribution of changes in lung impedance or a patient, which correlates with regional distribution of lung volume and regional distribution of Tidal Volume, that is the volume or air that a patient receives in a respiratory cycle. A plethysmogram is a waveform of the sum of impedance changes in a region of interest, plotted over time. Therefore, the plethysmogram waveform correlates with regional lung volume. In some embodiments, the lung volume and ventilation distribution of the patient are measured using other known processes and/or devices such as ultrasonic flow metering, MRI, tracer gas, etc.

EIT is an imaging technique involving the positioning electrodes via an electrode belt placed around a region of a patient's body (e.g., around the patient's chest for imaging of a lung), injecting electrical excitation signals through a pair of electrodes, and measuring the induced response signals detected by the other electrodes of the electrode belt. As a result, the EIT system may generate an image based on the voltage measurements indicating estimated impedance values. In contrast with other imaging techniques, EIT is non-invasive and does not have certain exposure risks that might limit the number and frequency of monitoring actions (e.g., as with techniques such as X-rays). As a result, EIT is suitable for continuously monitoring the condition of the patient, with particular application to monitoring the patient's lungs as the measurements may be used to determine respiratory and hemodynamic parameters of the patient and monitor a real-time two-dimensional image.

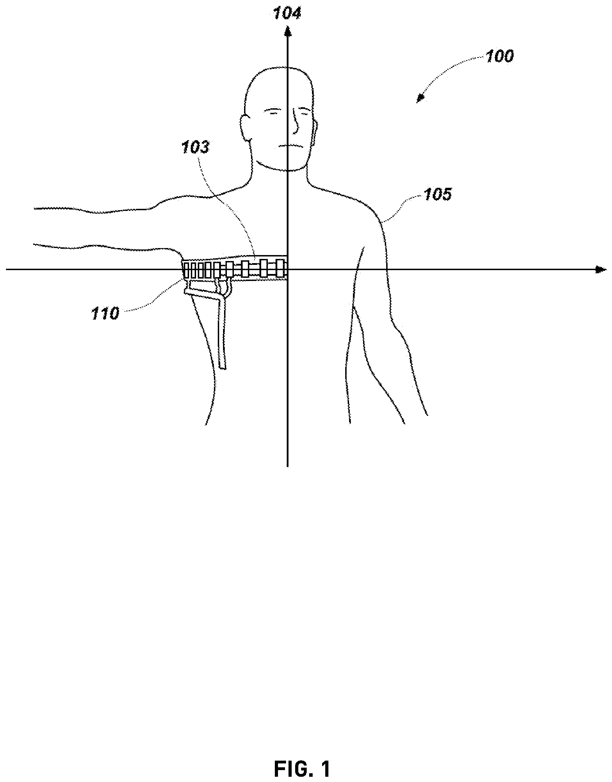

FIG. 1 is a schematic diagram of a portion of an EIT system 100 showing a plurality of electrodes 110 positioned around a region of interest (e.g., thorax) of a patient 105. The electrodes 110 of the EIT system 100 may be physically held in place by an electrode belt 103. The placement of the electrodes 110 may be transverse to a cranial caudal axis 104 of the patient. Although the electrodes 110 are shown in FIG. 1 as being placed only partially around the patient 105, electrodes 110 may by placed around the entire patient 105 depending on the specific region of interest available or desired for measurement. The electrodes 110 may be coupled to a computing system configured to control the operation of the electrodes 110 and perform reconstruction of the EIT image.

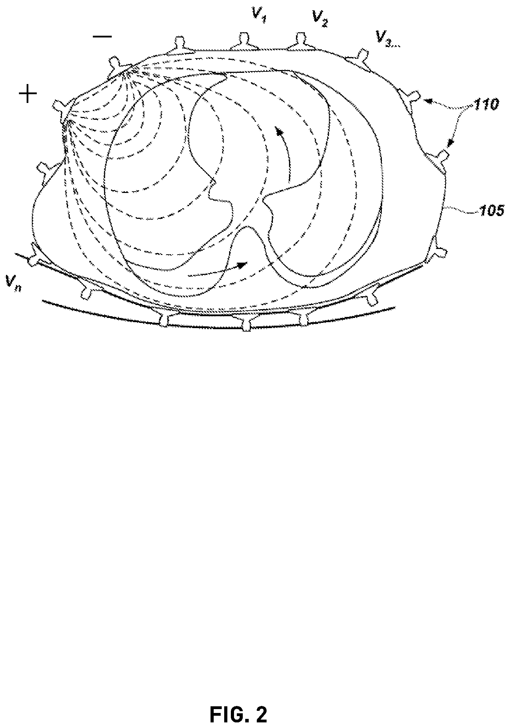

FIG. 2 is a schematic diagram showing a cross-section of the thorax of the patient 105 along the plane of the electrodes. A voltage may be applied to a pair of electrodes 110 (shown by the electrodes having a + and - symbol) to inject an excitation current into the patient between an electrode pair. As a result, voltages (e.g., $V_1, V_2, V_3 ... V_n$) may be detected by the other electrodes and measured by the EIT system 100. Current injection may be performed for a measurement cycle according to a circular pattern using different electrode pairs to generate the excitation current.

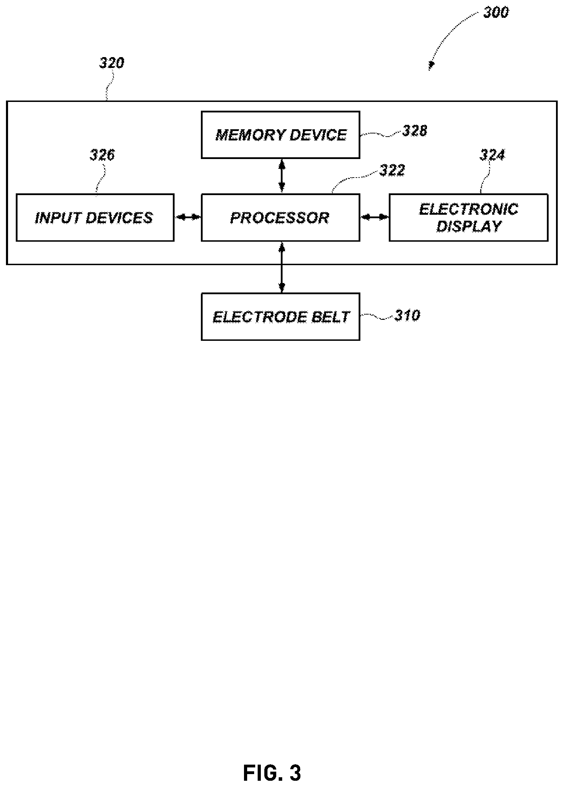

FIG. 3 is a schematic block diagram of an EIT system 300 according to an embodiment of the disclosure. The EIT system 300 may include an electrode belt 310 operably coupled with a data processing system 320. The electrode belt 310 and the data processing system 320 may be coupled together via a wired connection (e.g., cables) and/or may have communication modules to communicate wirelessly with each other. The data processing system 320 may include a processor 322 operably coupled with an electronic display 324, input devices 326, and a memory device 328. The electronic display 324 may be constructed with the data processing system 320 into a singular form factor for an EIT device coupled with the electrode belt 310. In some embodiments, the electronic display 324 and the data processing system 320 may be separate units of the EIT device coupled with the electrode belt 310. In yet other embodiments, an EIT system 300 may be integrated within another host system configured to perform additional medical measurements and/or procedures, in which the electrode belt 310 may couple to a port of the host system already having its own input devices, memory devices, and electronic display. As such, the host system may have the EIT processing software installed therein. Such software may be built into the host system prior to field use or updated after installation.

The processor 322 may coordinate the communication between the various devices as well as execute instructions stored in computer-readable media of the memory device 328 to direct current excitation, data acquisition, data analysis, and/or image reconstruction. As an example, the memory device 328 may include a library of finite element meshes used by the processor 322 to model the patient's body in the region of interest for performing image reconstruction. Input devices 326 may include devices such as a keyboard, touch screen interface, computer mouse, remote control, mobile devices, or other devices that are configured to receive information that may be used by the processor 322 to receive inputs from an operator of the EIT system 300. Thus, for a touch screen interface the electronic display 324 and the input devices 326 receiving user input may be integrated within the same device. The electronic display 324 may be configured to receive the data and output the EIT image reconstructed by the processor for the operator to view. Additional data (e.g., numeric data, graphs, trend information, and other information deemed useful for the operator) may also be generated by the processor 322 from the measured EIT data alone, or in combination with other non-EIT data according to other equipment coupled thereto. Such additional data may be displayed on the electronic display 324.

The EIT system 300 may include components that are not shown in the figures, but may also be included to facilitate communication and/or current excitation with the electrode belt 310 as would be understood by one of ordinary skill in the art, such as including one or more analog to digital converter, signal treatment circuits, demodulation circuits, power sources, etc.

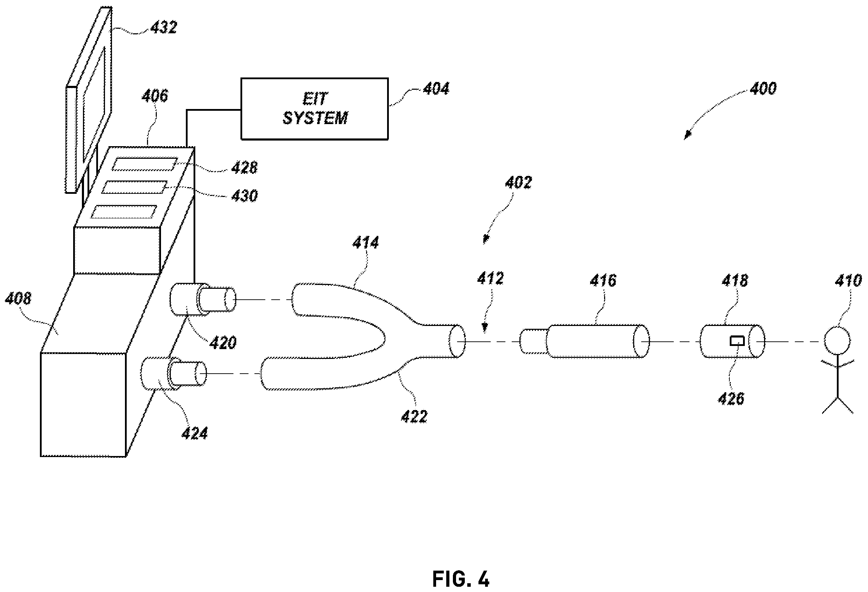

FIG. 4 depicts a schematic view of a system 400 for mechanically ventilating a patient. For instance, the system 400 includes a ventilator system 402, an EIT system 404, and a controller 406 for operating the system 400. The ventilator system 402 and the EIT system 404 are operably coupled to the controller 406.

In some embodiments, the ventilator system 402 includes a mechanical ventilator 408 that provides respiratory support or respiratory assistance to a patient 410. For instance, the mechanical ventilator 408 may provide a flow of medical gas, which may include one or more of air, oxygen, nitrogen, and helium. In some embodiments, the flow of medical gas may further include additives such as aerosol drugs or anesthetic agents. The ventilator system 402 may further include a breathing circuit 412, an inspiratory limb 414, a patient limb 416, and a patient connection 418. In some embodiments, the mechanical ventilator 408 may provide a flow of medical gas to the breathing circuit 412 through the inspiratory limb 414, which is connected to an inspiratory port 420 of the mechanical ventilator 408. The medical gas may flow (e.g., travel) through the inspiratory limb 414 and into the patient limb 416 of the breathing circuit 412. Accordingly, the mechanical ventilator 408 may provide the medical gas to the patient 410 through the patient connection 418.

Expired gases from the patient 410 may be delivered back to the mechanical ventilator 408 through the patient connection 418 and the patient limb 416. In some embodiments, the expired gases may be directed into an expiratory limb 422 of the breathing circuit 412 via one or more valves (e.g., check valves). For instance, the ventilator system 402 may further include a plurality of check valves, which may be placed at various points along the breathing circuit 412 such as to only permit medical gas flow in a desired direction along the appropriate pathway towards or away from the patient 410.

Additionally, the expired gases may be returned to the mechanical ventilator 408 through an expiratory port 424 of the mechanical ventilator 408.

In some embodiments, the expiratory port 424 includes a controllable flow valve that is adjustable to regulate the pressure within the breathing circuit 412. Adjusting the flow valve may create a back pressure, which is applied to the patient 410 during exhalation to create a positive end expiratory pressure ("PEEP"). Accordingly, the system 400 may include any conventional system for providing PEEP therapy to a patient 410. Additionally, other systems and configurations as recognized by one of ordinary skill in the art fall within the scope of the present disclosure.

The ventilator system 402 may further include one or more gas monitoring sensors 426. In some embodiments, the one or more gas monitoring sensors 426 may be disposed within the patient connection 418 of the breathing circuit 412. In alternative embodiments, the one or more gas monitoring sensors 426 may be fluidly connected to any other component of the breathing circuit 412 or the ventilator system 402. In some embodiments, the gas monitoring sensor 426 includes one or more of a pressure, a flow, and a gas concentration sensor. As is described in further detail below, the controller 406 and the mechanical ventilator 408 may utilize the one or more gas monitoring sensors 426 to monitor and ultimately, control the operation of the mechanical ventilator 408 and provide information (e.g., feedback to a user (e.g., clinician)). The one or more gas monitoring sensors 426 may include, without limitation, any conventional gas sensor(s).

The EIT system 404 may include any of the EIT systems described above with reference to one or more of FIGS. 1-3, and may operate according to any of the embodiments described above. The EIT system 404 may include, without limitation, a conventional EIT system. Additionally, as noted above, the EIT system 404 may be operably coupled to the controller 406 and may provide information to controller 406 regarding measurements performed by the EIT system 404. In some embodiments, the EIT system 404 is completely independent of the ventilator system 402. In one or more embodiments, the EIT system 404 also has a respective electronic display and input devices separate from displays and/or input devices of the ventilator system 402.

The controller 406 may include a processor 428 coupled to a memory 430 and an input/output component 432. The processor 428 may comprise a microprocessor, a field-programmable gate array, and/or other suitable logic devices. The memory 430 may include volatile and/or nonvolatile media (e.g., ROM, RAM, magnetic disk storage media, optical storage media, flash memory devices, and/or other suitable storage media) and/or other types of computer-readable storage media configured to store data. The memory 430 may store algorithms and/or instructions for operating the ventilator system 402 and the EIT system 404, to be executed by the processor 428. For example, the controller 406 may include the data processing system 320 described above with reference to FIG. 3. In some embodiments, the processor 428 is operably coupled to send data to a computing device operatively coupled (e.g., over the Internet) to the controller 406, such as a server or personal computer. The input/output component 432 may include a display, a touch screen, a keyboard, a mouse, and/or other suitable types of input/output devices configured to accept input from and provide output to an operator.

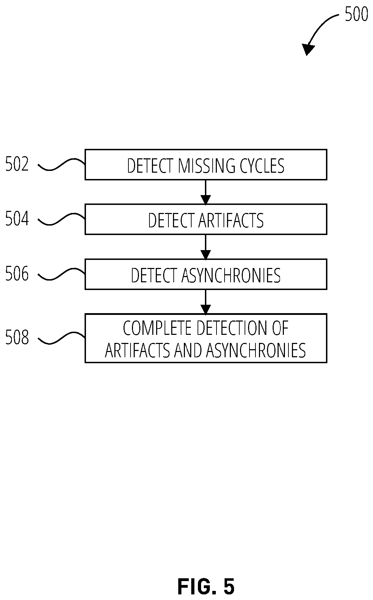

Referring still to FIG. 4, as is described in greater detail below with reference to FIGS. 5-13, the system 400 may utilize the ventilator system 402 to apply levels of PEEP to a patient in determining a potential for recruitment value of a patient's lung capacity (e.g., collapsed parenchyma) during an RAM. PEEP increases a base line pressure within the patient's respiratory system such that natural exhalation by the patient maintains a higher airway pressure than respiration without PEEP therapy. Conventional PEEP pressures range up to 40 cmH$_2$O, although higher PEEP pressures may also be used. High PEEP refers to PEEP therapy applied above 10 cmH$_2$O, and more specifically, 10-30 cmH$_2$O. Low PEEP refers to PEEP pressures below 10 cmH$_2$O and which are often applied at 5-8 cmH$_2$O.

In some embodiments, the effects of applying PEEP to a patient are measured by measuring a volume of the patient's lungs in response to the application of PEEP. After PEEP application, the volume of the patient's lungs is measured as the end expiratory lung volume (EELV) and is measured for a particular PEEP pressure applied to the patient. In some embodiments, EELV is measured at zero PEEP ("ZEEP"). The measurement of EELV at ZEEP is referred to herein as functional residual capacity (FRC) and is a measurement of the volume of air that remains in the lungs at the end of natural expiration. In some embodiments, when utilizing the EIT system 404 to measure and/or determine EELV, the FRC can, in some instances, be considered to be zero, such that the FRC does not affect certain calculations. In view of the foregoing, EELV is FRC plus lung volume increased by the applied PEEP.

Increases in EELV associated with the application of PEEP come from two physiological sources. A first physiological source of volume increase results from the application of additional pressure on the lung tissue. Applying additional pressure on lung tissue causes the lungs and already-opened alveoli to distend (e.g., swell due to pressure inside the lungs), creating more lung volume. Distending the lungs presents risks to a patient in the form of volutrauma (i.e., local over distention of normal alveoli), which damages the lungs. Volutrauma can result in medical complications with the patient similar to Acute Respiratory Distress Syndrome (ARDS). A second physiological source of increased lung volume is the "recruitment" of alveoli in a process known as "pop-open," where an internal volume of one alveolus suddenly jumps from a zero volume (e.g., collapsed) to a volume attained by neighboring alveoli (e.g., neighboring units). As is known in the art, alveoli are the air sacs within the lungs that promote gas exchange with a patient's blood. Some alveoli, particularly diseased or distressed alveoli, collapse when the pressure within the lungs falls too low, with the alveoli (e.g., unit) attaining or reaching a zero volume. Applying PEEP to a patient (e.g., applying a PEEP therapy) can maintain a minimum airway pressure within the lungs and, in some instances, cause alveoli (e.g., collapsed alveoli) to remain open.

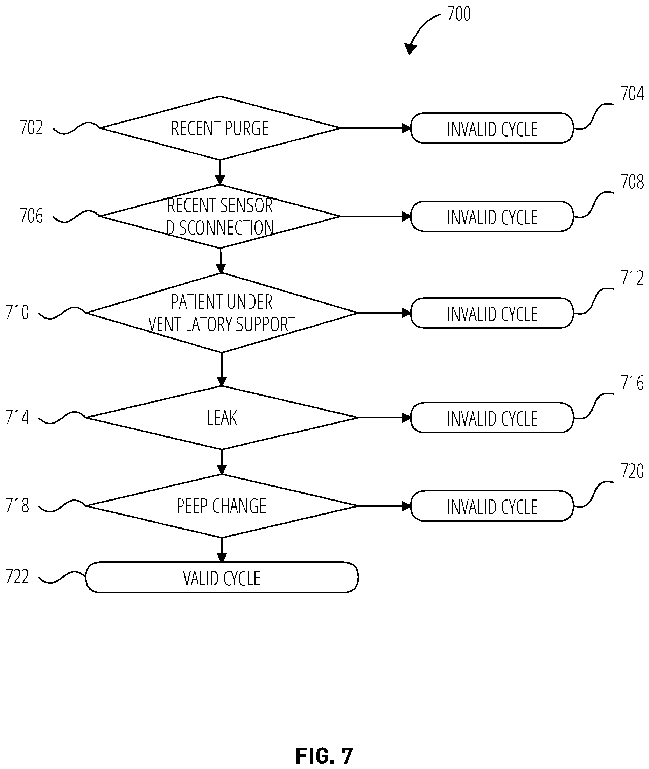

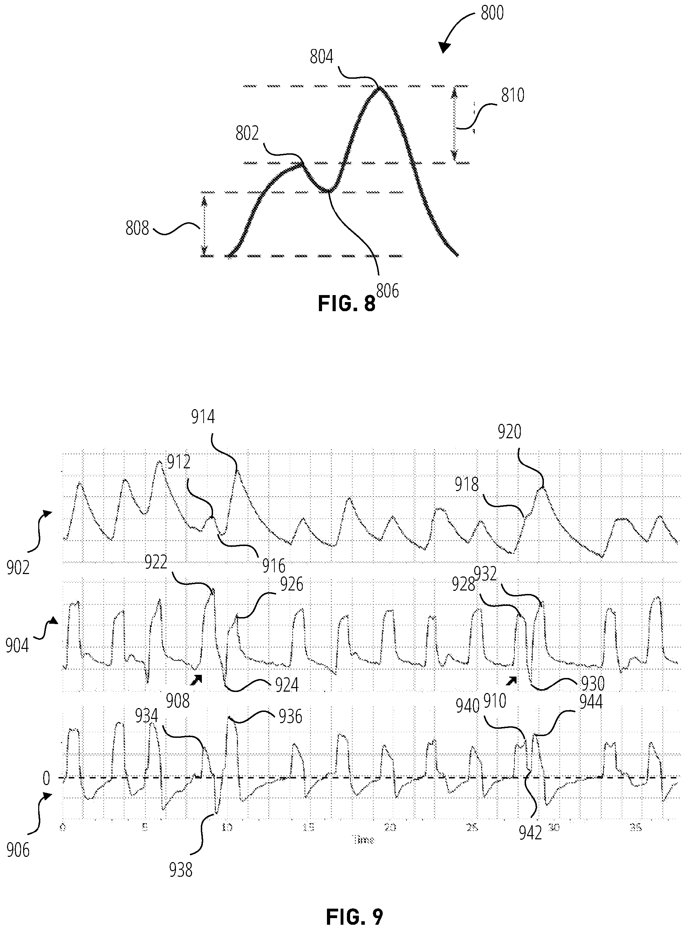

The alveoli can only generate some gas exchange when the alveoli are open. Therefore, in order to maintain some gas exchange when PEEP is insufficient, there is a need of higher driving inspiratory pressure to hyperventilate the already opened alveoli in order to compensate for the lack of function of the closed alveoli (e.g., units). Increased respiratory force causes greater distension on the remaining open alveoli, which may result in further damage to the lung tissue. In contrast, when PEEP is sufficient, the gas exchange is shared among most alveoli (e.g., units), which decreases the driving inspiratory pressure and, in some instances, lung damage. The recruitment of alveoli, therefore, also increases EELV, which correspond to the extra-volume generated by the pop-open of multiple alveolar units. The extra-volume created by opened alveolar units is known as the volume-gain (or vertical displacement in a pressure-volume plot) of the lung at a certain pressure. In a graph having two pressure-volume plots representing lung inflation with a first plot representing the inflation of the lung in a case of no recruitment, and with a second plot representing the conditional inflation of the lung when lung alveoli (e.g., units) were opened since a beginning of inflation, a vertical distance between the two curves (see FIGS. 7 and 8) represents a volume-gain caused by recruitment.

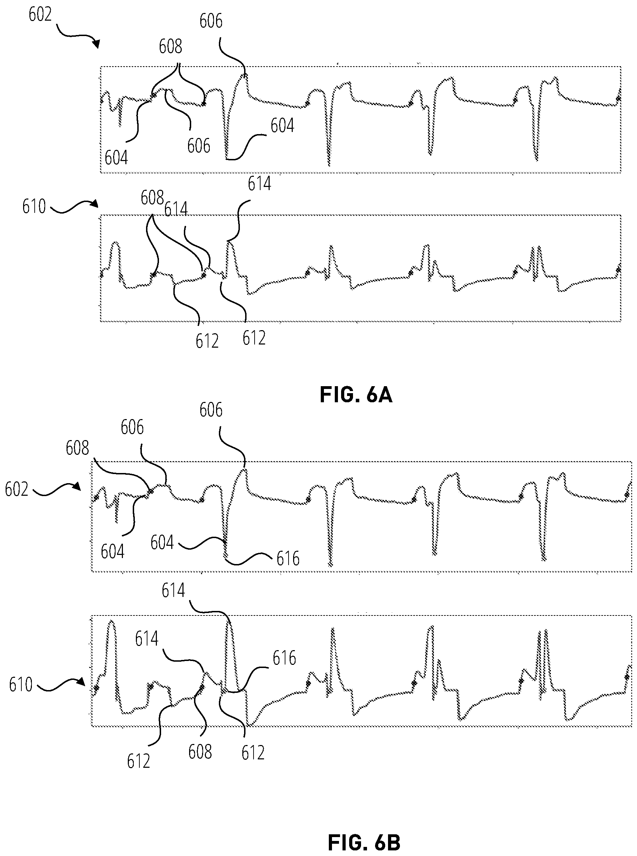

FIG. 5 illustrates a flow chart representative of a process of evaluating respiratory cycles 500. The individual process actions are described in further detail below with respect to FIG. 6A through FIG. 11. The process actions may be performed by a controller, such as controller 406 (FIG. 4) using mechanical ventilation data received from a mechanical ventilator (e.g., mechanical ventilator 408 (FIG. 4)), gas flow or gas pressure sensors (e.g., gas monitoring sensors 426 (FIG. 4)), and an EIT system (e.g., EIT system 404 (FIG. 4)). The data may include gas flow values over time (e.g., gas flow wave forms), gas pressure values over time (e.g., gas pressure wave forms), ventilator cycles, inspiration signals (e.g., inspiration event triggers or inspiration event trigger points), expiration signals (e.g., expiration event triggers or expiration event trigger points), EIT images and the underlying data (e.g., plethysmographic data, lung impedance values over time, lung volume values over time, lung volume waveforms, plethysmographs, regional lung volume values over time), etc.

A combination of the mechanical ventilator and the gas monitoring sensors may provide a gas flow wave form and a gas pressure wave form to the controller. The gas monitoring sensors may also provide inspiration triggers and expiration triggers. The inspiration triggers and expiration triggers may be trigger points that indicate when the patient switches from breathing in to breathing out or from breathing out to breathing in. In some embodiments, the inspiration triggers and expiration triggers are transmitted to the mechanical ventilator to trigger the mechanical ventilator to provide pressurized air to the patient (an inspiration event) or to remove pressure and allow air to leave the patient's lungs or provide a negative pressure to draw air out of the patient's lungs (an expiration event). The gas monitoring sensors may determine that there is an inspiration trigger point or expiration trigger point based on a sudden change in one or more of gas flow, gas pressure, or flow volume in the breathing circuit between the mechanical ventilator and the patient. The inspiration triggers and expiration triggers are used to define a breathing cycle. The breathing cycle may begin at the beginning of an inspiration event and end at the end of a subsequent expiration event or at the transition from the expiration event to the next inspiration event.

The process of evaluating respiratory cycles 500 may begin by determining if any trigger events (e.g., inspiration triggers or expiration triggers) were missed, such that cycles were missed or mis-identified in act 502. Asynchronous events between the mechanical ventilator and the patient may result in one or more trigger events being missed by the gas monitoring sensors. Thus, the process may begin by analyzing the gas pressure wave forms and the gas flow waveforms and identifying trigger events that are not included in the inspiration triggers and expiration triggers from the gas monitoring sensors. The identification of missing trigger events is described in further detail below with reference to FIG. 6A and FIG. 6B.

After identifying missing trigger events, the process of evaluating respiratory cycles 500 may search for artifacts in act 504. As used herein, an "artifact" is an event that will cause (e.g., result in, effectuate) inaccurate readings, such as a sensor disconnection, a change in parameters (e.g., pressure, PEEP, flow, etc.), purge, aspiration, etc. If an artifact is detected, the associated breathing cycle is labeled as an invalid cycle. This may substantially prevent the controller from mis-identifying an asynchrony during an artifact event. The detection of artifacts is described in further detail below with reference to FIG. 7.

After identifying artifacts in act 504, the process of evaluating respiratory cycles 500 searches the data for asynchronies in act 506. As used herein, an "asynchrony" is a situation where the mechanical ventilator and the patient are not synchronized. Asynchronies may include cojoined cycles, double triggers, reverse triggers, breath stacking, breath stacking volumes, trapped volumes, and/or stacked volumes. Asynchronies may be identified by comparing lung volume data from the EIT system to the gas pressure wave forms and gas flow waveforms from the gas monitoring sensors. The detection of different asynchronies is described in further detail below with reference to FIG. 8 through FIG. 11.

After identifying the artifacts in act 504 and the asynchronies in act 506, the controller combines the artifacts and asynchronies in act 508. The controller may display the artifacts and asynchronies as a report on a display, such as the input/output component 432 or the electronic display 324. The controller may also create an index for a patient including one or more of the number of asynchronies, the number of artifacts, the types of each of the asynchronies and artifacts, the frequency of each occurrence, etc.

In some embodiments, the controller provides recommendations for parameter adjustments to the mechanical ventilator, such as adjustments to PEEP, sensitivity adjustments, etc. In other embodiments, the controller sends signals to the mechanical ventilator configured to adjust the one or more parameters of the mechanical ventilator based on the asynchronies detected in act 506. Different types of asynchronies may be corrected through different adjustments to the mechanical ventilator settings, such as PEEP and Plateau pressures, inspiratory and expiratory times, ventilation modes, sensitivity levels, and respiratory rates.

FIGS. 6A and 6B illustrate pressure waveforms 602 and flow waveforms 610 aligned in time. FIG. 6A illustrates the pressure waveform 602 and the flow waveform 610 before analyzing the pressure waveform 602 and the flow waveform 610 for missing triggers and FIG. 6B illustrates the pressure waveform 602 and the flow waveform 610 after inserting the missing triggers.

The pressure waveform 602 includes peaks 606 corresponding to the high pressure points and valleys 604 corresponding to low pressure points. Trigger points 608 detected by the gas monitoring sensors are also plotted along the pressure waveform 602. The trigger points 608 illustrated in FIGS. 6A and 6B correspond to inspiration trigger points, which occur proximate valleys 604 in the pressure waveform 602, due to a reduction in pressure caused by the patient inhaling.

The flow waveform 610 also includes peaks 614 corresponding to high gas flow and valleys 612 corresponding to low gas flow. The trigger points 608, corresponding to inflection trigger points, are also plotted on the flow waveform 610.

As discussed above, the gas monitoring sensors may fail to recognize trigger points. In some cases, the missed trigger points correspond to asynchronies between the mechanical ventilator and the patient, such as double triggering events or reverse triggering events. To detect missed triggers, the controller may apply waveform filtering to the pressure waveform 602 and the flow waveform 610. The waveform filtering may be a moving average filter that averages the respective pressure waveform 602 or flow waveform 610 at each point over a limited number of frames, such as within a range of from about 2 frames to about 20 frames, from about 2 frames to about 10 frames, or about 5 frames.

After filtering the waveforms, the controller may identify potentially unidentified trigger points. For example, the controller may identify potentially unidentified trigger points where the flow value is greater than threshold flow, the flow difference between two adjacent points on the flow waveform 610 is greater than a threshold amount, and the pressure difference between two adjacent points on the 602 is greater than a threshold amount. The threshold flow value may be within a range of from about 0.2 L/s to about 0.7 L/s, such as about $1.5e^{-1}$ L/s. The threshold flow difference between adjacent points on the flow waveform 610 may be within a range of from about 0.0 L/s to about 0.1 L/s. The threshold pressure difference between adjacent points on the pressure waveform 602 may be within a range of from about 0.8 $cmH_2O$ to about 1.1 $cmH_2O$, such as about $2.6e^{-1}$ $cmH_2O$.

The controller may then compare the potentially unidentified trigger points to the trigger points 608 that were previously identified by the gas monitoring sensors. The controller may then remove potentially unidentified trigger points that are close in proximity to previously identified trigger points 608. For example, a potentially unidentified trigger point occurring within a short period of time from one of the previously identified trigger points 608 is likely the same trigger point. Therefore, a potentially unidentified trigger point occurring less than about 200 milliseconds (ms), such as less than about 100 ms, from a previously identified trigger point 608 may be removed from consideration. The controller may also remove potentially unidentified trigger points occurring close in time to other potentially unidentified trigger points. For example, potentially unidentified trigger points occurring within about 200 ms, such as within about 100 ms of another unidentified trigger point may be removed from consideration.

The potentially unidentified trigger points remaining after comparing the potentially unidentified trigger points to the previously identified trigger points 608 may then be traced to the closest local minimum pressure (e.g., the closest valley 604 in the pressure waveform 602). The rate of flow at each of the potentially unidentified triggers may also be considered. For example, at a trigger point, the flow should be changing direction. Therefore, the flow should be low at a trigger point. Thus, the controller may eliminate potentially unidentified trigger points where the gas flow is greater than about 0.1 L/s, such as greater than about 0.2 L/s. In some embodiments, the controller also considers a flow volume at the potentially unidentified trigger points. For example, the controller may eliminate potentially unidentified trigger points where the flow volume is below an expected amount, such as less than about 1.2 milliliters/kilogram (mL/kg), or less than about 1 mL/kg.

The remaining potentially unidentified trigger points may then be plotted on the pressure waveform 602 and the flow waveform 610 as missed trigger points 616 as illustrated in FIG. 6B.

The controller may similarly detect unidentified expiration trigger points that correspond to the unidentified inspiration trigger points. The controller may follow similar steps to detect unidentified expiration trigger points to the steps used to detect the unidentified inspiration trigger points.

FIG. 7 illustrates a flow chart 700 representative of a decision process for determining if an identified cycle, including both an inspiration trigger point and an expiration trigger point, is a valid cycle. As discussed above, artifacts, such as a sensor disconnection, a change in parameters (e.g., pressure, PEEP, flow, etc.), purge, aspiration, etc., may cause erroneous readings. Therefore, the controller may determine if an artifact is associated with a cycle to determine if the portion of the pressure waveform 602, the portion of the flow waveform 610, and other data associated with the cycle is valid. If an artifact is detected, the associated breathing cycle is labeled as an invalid cycle through the decision process illustrated in FIG. 7.

In decision 702, the controller may determine if a recent purge occurred. For example, the controller may determine if a purge occurred during the cycle at issue or the cycle immediately preceding the cycle at issue. If the controller determines that a recent purge event occurred, the cycle at issue is determined to be invalid in act 704.

In decision 706, the controller may determine if a sensor was recently disconnected. For example, the controller may determine if a sensor was disconnected during the cycle at issue or the cycle immediately preceding the cycle at issue. If the controller determines that a sensor was recently disconnected the cycle at issue is determined to be invalid in act 708.

In decision 710, the controller may determine if the patient is under ventilatory support. For example, if the mechanical ventilator is operating at a higher pressure, the ventilator is breathing for the patient rather than assisting a breathing patient. For example, if an average pressure is greater than 4 $cmH_2O$, the patient may be determined to be under ventilatory support. When the ventilator is breathing for the patient asynchronies will not occur because the mechanical ventilator is not relying on signals from the patient to move through the breathing cycle. If the controller determines that the patient is under ventilatory support, the cycle is determined to be invalid in act 712.

In decision 714, the controller may determine if there is a leak in the system. The controller may monitor a gas volume for each cycle. The controller may analyze multiple cycles, such as within a range of from about 4 cycles to about 16 cycles, or from about 8 cycles to about 12 cycles. If the controller determines that a change in gas volume occurs across the multiple cycles, the controller may determine that a leak has been detected. If a leak is detected, the cycle is determined to be invalid in act 716.

In decision 718, the controller may determine if a pressure in the system, such as a PEEP pressure has recently changed. For example, the controller may determine if the pressure in the system changed during the cycle of interest or in a preceding cycle. The preceding cycle may include cycles in the range from the immediately preceding cycle to the preceding ten cycles, such as the preceding two cycles to the preceding eight cycles. If a pressure change has occurred in a preceding cycle, the cycle is determined to be invalid in act 720.

If none of the artifacts listed and discussed above are detected, the cycle at issue may be determined to be valid in act 722. The valid cycle may be further analyzed in the subsequent acts of the process of evaluating respiratory cycles 500 (FIG. 5). The controller may then determine if the cycles include an asynchronous event, such as cojoined cycles, double triggers, reverse triggers, breath stacking, breath stacking volumes, trapped volumes, or stacked volumes.

FIG. 8 illustrates an exemplary view of a conjoined cycle in a lung volume waveform 800. The lung volume waveform 800 may be provided by an EIT system, such as the EIT system 404 (FIG. 4). A conjoined cycles occurs when a single patient effort triggers two subsequent respiratory cycles with shorter expiratory time between them (double triggering) or when a first cycle is triggered by the ventilator (without muscle effort) and a second is triggered by the patient's effort (reverse triggering). Conjoined cycles may result in breath-stacking. Breath-stacking is an unintentional high tidal volume which jeopardizes the low tidal volume (VT) protective strategies and increases the risk of lung injury. Breath stacking may also cause hyperinflation of the lungs, thus leading to hypoxemia and hypercapnia.

The lung volume waveform 800 of FIG. 8 includes a first peak 802 and a second peak 804 separated by an intermediate valley 806. The intermediate valley 806 represents the short expiratory time between the two respiratory cycles, characterized by the first peak 802 and the second peak 804. The intermediate valley 806 may define a trapped volume 808. The trapped volume 808 is the volume of air within the lungs that was not expelled during the short expiratory time. The second peak 804 defines a stacked volume 810, which is the additional volume of air added to the lungs in excess of what a conventional cycle would add. The stacked volume 810 is the volume of air that has the potential to injure the lungs.

FIG. 9 illustrates a lung volume waveform 902, a pressure waveform 904, and a flow waveform 906 demonstrating a double trigger event 908 and a reverse trigger event 910. The lung volume waveform 902 illustrates changes in the volume of the patient's lungs over time as measured by an EIT system. The pressure waveform 904 illustrates increases and decreases in pressure in the breathing circuit as measured by gas monitoring sensors or a mechanical ventilator. The flow waveform 906 illustrates changes in a flow velocity within the breathing circuit as measured by gas monitoring sensors or a mechanical ventilator. The flow waveform 906 also indicates a flow direction, for example, a flow velocity less than 0 is in an expiratory flow direction away from the patient and a flow velocity greater than 0 is in an inspiratory flow direction toward the patient.

As discussed above, the double trigger event 908 occurs when a single patient effort triggers two subsequent respiratory cycles from the mechanical ventilator with shorter expiratory time between them. As illustrated the lung volume waveform 902 includes a smaller first peak 912 and a larger second peak 914 separated by an intermediate valley 916 representative of the short expiratory time. The double trigger event 908 may result in a significant increase in lung volume relative to the conventional cycles. The pressure waveform 904 illustrates a larger first peak 922 and a smaller second peak 926 separated by an intermediate valley 924. The flow waveform 906 illustrates a first peak 934 and a second peak 936 separated by a valley 938 where the flow changes from inspiratory flow at the first peak 934 to expiratory flow at the valley 938 and back to inspiratory flow at the 936.

The reverse trigger event 910 occurs when a first cycle is triggered by the ventilator without the patient's effort and a second cycle is triggered by the patient's effort. As illustrated the lung volume waveform 902 includes a first peak 918 and a second peak 920 but there is no intermediate valley as there is no expiratory time between the two inspirations. Similar to the double trigger event 908, the reverse trigger event 910 may result in a significant increase in the lung volume relative to the conventional cycles due to the two inspirations without an intervening expiration. The pressure waveform 904 illustrates a smaller first peak 928 and a larger second peak 932 separated by an intermediate valley 930. The flow waveform 906 illustrates a first peak 940 and a second peak 944 separated by a valley 942. In the reverse trigger event 910 the flow does not change from inspiratory flow to expiratory flow, instead the flow velocity reduces at the valley 942 but continues in an inspiratory direction.

To detect the different asynchronous events, the controller may begin by applying a filter to the lung volume waveform 902, the pressure waveform 904, and the flow waveform 906. The pressure waveform 904 and the flow waveform 906 may be filtered through a moving average filter that may average the data of the respective waveform 904, 906 across multiple frames, such as in the range of 5 frames to about 20 frames or about 10 frames. The lung volume waveform 902 may be filtered using a high-pass filter, such as a high-pass Butterworth filter-off frequency in a range from about 0.01 Hz and about 0.03 Hz, such as about 0.02 Hz.

After filtering the lung volume waveform 902, the pressure waveform 904, and the flow waveform 906, the controller may analyze features of each cycle in the waveforms 902, 904, 906 to determine if a conjoined cycle occurred. For example, the controller may determine a trapped volume 808 during each cycle. If the trapped volume 808 is greater than a threshold volume the cycle may be identified for further analysis. The threshold volume may be in a range from about 0.5 mL/kg to about 2 mL/kg, such as 1 mL/kg. The controller may also determine if an expiratory time between two cycles, such as the flagged cycles, is below a threshold time. The threshold time may be in a range from about 0.5 s to about 2 s, such as about 1 s. The controller may also compare the expiratory time between the two cycles to previous expiratory times. For example, the controller may calculate a value G using the following formula:

$$G = 1 - \min\left\{ \frac{t_{exp}}{\bar{t}_{exp}}, 1 \right\}$$

Where $\bar{t}_{exp}$ is a trimmed mean of the several past cycles, such as the past ten cycles, the past fifteen cycles, or the past twenty cycles. If the calculated value G is greater than a threshold value, the controller determines that the associated two cycles are a conjoined cycle. The threshold value may be within a range of from about 0.3 to about 0.8, such as about 0.5. A cycle or two cycles meeting the above thresholds is defined as an asynchronous cycle.

Once an asynchronous cycle is identified, the controller may then classify the type of asynchrony. For example, the distinction between whether a cycle is double triggering or reverse triggering depends on the nature of the first cycle: if the first cycle was triggered by the patient muscular effort, it is labeled as double triggering; if the first cycle was triggered by the ventilator, it is labeled as reverse triggering.

The controller may determine an estimate of the muscular effort of the patient through the following formula:

$$P_{EST} = \frac{1}{C_{max}} V + R_{min} F - P$$

Where:

$$C_{max} = 80 \frac{mL}{cmH_2O}$$

$$R_{min} = 5 \frac{cmH_2O \cdot s}{L}$$

Rough muscular effort estimate is used to evaluate a grade . First a window (W) is defined using the following formula:

$$W = \{P_{est}(t_i) | t_i \in [t_{ins} - 0.2s, t_{ins} + 0.2s]\},$$

G is then estimated using the following relationships:

if $f(W) = t_{ins}$ - 20 then G = 0, where $t_0$ is the point of trigger;
if $f(W) \neq t_{ins}$ - 20, then $G = \max(W) - \min(W_1)$, where

$$W_1 = \{P_{est}(t_i) | t_i \in [t_{ins} - 20, \ f(W)]\}$$

After G is estimated through the above relationships, G may be used to determine which type of asynchronous event is associated with the cycle(s) at issue. For example, if G is greater than a threshold value, the controller may determine that the event was triggered by the patient and is double triggering. On the other hand, if G is less than the threshold value, the controller may determine that the event was triggered by the ventilator and is reverse triggering. For example, the threshold value may be set to 1.38. Thus, if G is greater than or equal to 1.38 the second cycle is determined to be a double triggering asynchrony. On the other hand, if G is less than 1.38, the cycle is determined to be reverse triggering.

Breath stacking is identified when a trapped volume 808 (FIG. 8) and a stacked volume 810 (FIG. 8) are greater than a threshold value. The threshold value may be set to a value that approximates the volume change of a conventional breath, such as within a range of from about 0.8 mL/kg to about 1.5 mL/kg, or about 1 mL/kg. Thus, if both the trapped volume 808 and the stacked volume 810 are greater than the threshold value the controller determines that the asynchronous event includes breath stacking.

One asynchronous event may include more than one asynchrony. For example, a double triggering event may also include a breath stacking event caused by the double triggering.

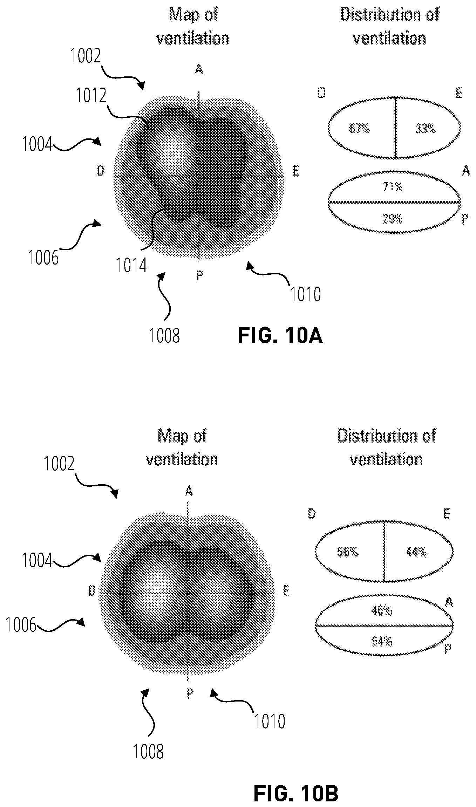

In some cases, an asynchronous event only affects a region of a patient's lungs. For example, as illustrated in FIG. 10A, an asynchronous event may cause one region of the patient's lungs to hyperinflate while not inflating other regions. Thus, monitoring global flow volume to the patient's lungs may not capture the event while monitoring regional volumes may provide the additional data to determine if such a condition exists.

As illustrated in FIG. 10A, pixel data from an EIT image 1002 may provide data regarding the volume of the patient's lungs in an anterior region 1004, a posterior region 1006, a right region 1008, and a left region 1010. The pixel data may facilitate defining a region of the patient's lungs as a hyper inflated region 1012 and another region of the patient's lungs as an under inflated region 1014. The pixel data may also facilitate determining the location of the hyper inflated region 1012 and the under inflated region 1014. The pixel data may be used in one or more of the processes described above to detect asynchronies. For example, regional stretching may be associated with reverse triggering asynchronous events. Thus, the controller may use pixel data both to detect asynchronous events and to classify the asynchronous events.

The embodiments of the disclosure provide a tool that may provide additional information to a physician regarding mechanically ventilated patients. The information may be used to reduce and/or prevent asynchronies between the mechanical ventilator and the patient, which may reduce the risk of ventilator related injuries. By evaluating the information in the manner discussed herein, embodiments of the disclosure may detect asynchronies that would be otherwise missed by an attentive physician. Furthermore, the embodiments of the disclosure may take the place of an experienced physician watching the monitors by detecting asynchronies and alerting the attending physician when an asynchrony is detected, which may reduce costs to a hospital and workloads of the attending physicians and staff.

The embodiments of the disclosure described above and illustrated in the accompanying drawing figures do not limit the scope of the invention, since these embodiments are merely examples of embodiments of the invention, which is defined by the appended claims and their legal equivalents. Any equivalent embodiments are intended to be within the scope of this disclosure Indeed, various modifications of the present disclosure, in addition to those shown and described herein, such as alternative useful combinations of the elements described, may become apparent to those skilled in the art from the description without departing from the scope of the invention as defined in the appended set of claims.

## Claims

1.   A system configured to detect asynchronous respiratory events; the system comprising:

a receiver (408) configured to receive ventilation data from at least one of a mechanical ventilator, an airway flow sensor (426), or an airway pressure sensors (426) and impedance data from an electrical impedance tomography device;
a processor (322);
a memory device (328) configured to store the ventilation data and the impedance data; and
a non-transitory computer readable medium storing instructions thereon that, when executed by the processor (322), cause the processor (322) to perform steps comprising:

retrieve a plethysmograph from the impedance data;
retrieve at least one of a flow waveform and a pressure waveform from the ventilation data;
compare the plethysmograph and the at least one of the flow waveform and the pressure waveform;
determine if an asynchronous event occurred based on comparing the plethysmograph and the at least one of the flow waveform and the pressure waveform;
classify the asynchronous event; and
provide a classification of the asynchronous event, wherein the instructions cause the processor (322) to:

determine a trapped volume during a cycle;
determine if an expiratory time between two cycles is below a threshold time; and
compare the expiratory time between the two cycles to previous expiratory times.

2. The system of claim 1, wherein the instructions cause the processor (322) to determine if the asynchronous event occurred if the trapped volume is greater than a threshold volume in a range of from about 0.5 mL/kg to about 2 mL/kg.

3. The system of claim 2, wherein the instructions cause the processor (322) to determine the asynchronous event occurred if:

   a stacked volume during a cycle is greater than the threshold volume;
   the expiratory time between the two cycles is below the threshold time; and
   the expiratory time between the two cycles is less than the previous expiratory times.

4. The system of any one of claims 1 through 3, wherein the threshold time is within a range of from about 0.5 second to about 2 seconds.

5. The system of any one of claims 1 through 3, wherein the previous expiratory times include expiratory times from at least four previous cycles.

6. The system of any one of claims 1 through 3, wherein the instructions cause the processor (322) to communicate a recommended adjustment to a user.

7. The system of any one of claims 1 through 3, wherein the instructions cause the processor (322) to send a signal providing an adjustment to the mechanical ventilator.

8. The system of any one of claims 1 through 3, wherein the instructions cause the processor (322) to determine if the respiratory cycle is valid by determining that no artifact affected the respiratory cycle.

9. The system of claim 8, wherein the artifact includes one of a sensor disconnection, a change in PEEP, a purge, and a leak.

10. A method of detecting an asynchronous respiratory event, the method comprising:

    retrieving an impedance data waveform from an electrical impedance tomography device;
    retrieving one or more of a flow waveform and a pressure waveform;
    aligning the impedance data waveform and the one or more of the flow waveform and the pressure waveform with respect to time;
    comparing the impedance data waveform and the one or more of the flow waveform and the pressure waveform;
    determining if an asynchronous respiratory event occurred based on comparing the impedance data waveform and the one or more of the flow waveform and the pressure waveform;
    classifying the asynchronous respiratory event; and
    providing information identifying the asynchronous respiratory event;
    wherein classifying the asynchronous respiratory event comprises determining if the asynchronous respiratory event is a double trigger event.

11. The method of claim 10, further comprising estimating an instant of a muscular effort of a patient.

12. The method of claim 11, wherein classifying the asynchronous respiratory event comprises determining if the instant of the muscular effort of the patient triggered a first cycle of the asynchronous respiratory event.


**Patentansprüche**

1. System, das so konfiguriert ist, dass es asynchrone Atmungsereignisse erkennt, wobei das System Folgendes umfasst:

   einen Empfänger (408), der konfiguriert ist, um Beatmungsdaten von mindestens einem von einem mechanischen Beatmungsgerät, einem Atemwegsströmungssensor (426) oder einem Atemwegsdrucksensor (426) und Impedanzdaten von einer elektrischen Impedanztomographievorrichtung zu empfangen;
   einen Prozessor (322);
   eine Speichervorrichtung (328), die zum Speichern der Beatmungsdaten und der Impedanzdaten konfiguriert ist;

und

ein nichtflüchtiges computerlesbares Medium, auf dem Anweisungen gespeichert sind, die, wenn sie von dem Prozessor (322) ausgeführt werden, den Prozessor (322) veranlassen, Schritte durchzuführen, die Folgendes umfassen:

Abrufen eines Plethysmographen aus den Impedanzdaten;
Abrufen, aus den Beatmungsdaten, von mindestens einer Strömungswellenform und einer Druckwellenform;
Vergleichen des Plethysmographen und der mindestens einen von der Strömungswellenform und der Druckwellenform;
Bestimmen, ob ein asynchrones Ereignis aufgetreten ist, auf Grundlage des Vergleichens des Plethysmographen und der mindestens einen von der Strömungswellenform und der Druckwellenform;
Klassifizieren des asynchronen Ereignisses; und
Bereitstellen einer Klassifizierung des asynchronen Ereignisses, wobei die Anweisungen den Prozessor (322) zu Folgendem veranlassen:

Bestimmen eines eingeschlossenen Volumens während eines Zyklus;
Bestimmen, ob eine Ausatmungszeit zwischen zwei Zyklen unter einer Schwellenzeit liegt; und Vergleichen der Ausatmungszeit zwischen den zwei Zyklen mit früheren Ausatmungszeiten.

2. System nach Anspruch 1, wobei die Anweisungen den Prozessor (322) veranlassen, zu bestimmen, ob das asynchrone Ereignis aufgetreten ist, wenn das eingeschlossene Volumen größer als ein Schwellenvolumen in einem Bereich von etwa 0,5 mL/kg bis etwa 2 mL/kg ist.

3. System nach Anspruch 2, wobei die Anweisungen den Prozessor (322) veranlassen, zu bestimmen, dass das asynchrone Ereignis aufgetreten ist, wenn:

ein gestapeltes Volumen während eines Zyklus größer als das Schwellenvolumen ist;
die Ausatmungszeit zwischen den zwei Zyklen unter der Schwellenzeit liegt; und
die Ausatmungszeit zwischen den zwei Zyklen kürzer als die vorherigen Ausatmungszeiten ist.

4. System nach einem der Ansprüche 1 bis 3, wobei die Schwellenzeit innerhalb eines Bereichs von etwa 0,5 Sekunden bis etwa 2 Sekunden liegt.

5. System nach einem der Ansprüche 1 bis 3, wobei die vorherigen Ausatmungszeiten Ausatmungszeiten von mindestens vier vorherigen Zyklen beinhalten.

6. System nach einem der Ansprüche 1 bis 3, wobei die Anweisungen den Prozessor (322) veranlassen, eine empfohlene Einstellung an einen Benutzer zu übermitteln.

7. System nach einem der Ansprüche 1 bis 3, wobei die Anweisungen den Prozessor (322) veranlassen, ein Signal zu senden, das eine Einstellung des mechanischen Beatmungsgeräts bewirkt.

8. System nach einem der Ansprüche 1 bis 3, wobei die Anweisungen den Prozessor (322) veranlassen, zu bestimmen, ob der Atemzyklus gültig ist, indem festgestellt wird, dass kein Artefakt den Atemzyklus beeinflusst hat.

9. System nach Anspruch 8, wobei das Artefakt eine Sensorabschaltung, eine PEEP-Änderung, eine Spülung und/oder ein Leck beinhaltet.

10. Verfahren zum Erkennen eines asynchronen Atmungsereignisses, wobei das Verfahren Folgendes umfasst: Abrufen einer Impedanzdatenwellenform von einer elektrischen Impedanztomographievorrichtung; Abrufen einer oder mehrerer von einer Strömungswellenform und einer Druckwellenform;

Ausrichten der Impedanzdatenwellenform und der einen oder der mehreren von der Strömungswellenform und der Druckwellenform in Bezug auf die Zeit;
Vergleichen der Impedanzdatenwellenform und der einen oder der mehreren von der Strömungswellenform und der Druckwellenform;
Bestimmen, ob ein asynchrones Atmungsereignis aufgetreten ist, auf Grundlage des Vergleichens der Impedanzdatenwellenform und der einen oder der mehreren von der Strömungswellenform und der Druckwellenform;
Klassifizieren des asynchronen Atmungsereignisses; und
Bereitstellen von Informationen, die das asynchrone Atmungsereignis identifizieren;
wobei das Klassifizieren des asynchronen Atmungsereignisses das Bestimmen umfasst, ob das asynchrone Atmungsereignis ein Doppelauslöserereignis ist.

11. Verfahren nach Anspruch 10, ferner umfassend das Schätzen eines Zeitpunkts einer Muskelanstrengung eines Patienten.

12. Verfahren nach Anspruch 11, wobei das Klassifizieren des asynchronen Atmungsereignisses das Bestimmen umfasst, ob der Zeitpunkt der Muskelanstrengung des Patienten einen ersten Zyklus des asynchronen Atmungsereignisses ausgelöst hat.

**Revendications**

1. Système configuré pour détecter des événements respiratoires asynchrones ; le système comprenant :

un récepteur (408) configuré pour recevoir des données de ventilation provenant au moins d'un ventilateur mécanique, d'un capteur de débit (426) des voies aériennes ou d'un capteur de pression (426) des voies aériennes et des données d'impédance provenant d'un dispositif de tomographie par impédance électrique ;

- un processeur (322) ;

un dispositif de mémoire (328) configuré pour stocker les données de ventilation et les données d'impédance ; et
un support non transitoire lisible par ordinateur stockant des instructions qui, lorsqu'elles sont exécutées par le processeur (322), amènent le processeur (322) à effectuer les étapes comprenant :

la récupération d'un pléthysmographe à partir des données d'impédance ;
l'extraction d'au moins une forme d'onde de débit et une forme d'onde de pression à partir des données de ventilation ;
la comparaison du pléthysmographe et d'au moins l'une des formes d'onde de débit et de pression ;
la détermination de la survenue d'un événement asynchrone en comparant le pléthysmographe et au moins l'une des formes d'onde de débit et de pression ;
la classification de l'événement asynchrone ; et
la fourniture d'une classification de l'événement asynchrone, les instructions amenant le processeur (322) à :

déterminer un volume piégé au cours d'un cycle ;
déterminer si un temps d'expiration entre deux cycles est inférieur à un temps de seuil ; et comparer le temps d'expiration entre les deux cycles aux temps d'expiration précédents.

2. Système selon la revendication 1, les instructions amenant le processeur (322) à déterminer si l'événement asynchrone s'est produit si le volume piégé est supérieur à un volume seuil dans une plage d'environ 0,5 mL/kg à environ 2 mL/kg.

3. Système selon la revendication 2, les instructions amenant le processeur (322) à déterminer que l'événement asynchrone s'est produit si :

un volume empilé au cours d'un cycle est supérieur au volume seuil ;
le temps d'expiration entre les deux cycles est inférieur au temps de seuil ; et
le temps d'expiration entre les deux cycles est inférieur aux temps d'expiration précédents.

4. Système selon l'une quelconque des revendications 1 à 3, le temps de seuil étant compris entre environ 0,5 seconde et environ 2 secondes.

5. Système selon l'une quelconque des revendications 1 à 3, les temps d'expiration précédents comprenant les temps d'expiration d'au moins quatre cycles précédents.

6. Système selon l'une quelconque des revendications 1 à 3, les instructions amenant le processeur (322) à communiquer un ajustement recommandé à un utilisateur.

7. Système selon l'une quelconque des revendications 1 à 3, les instructions amenant le processeur (322) à envoyer un signal fournissant un ajustement au ventilateur mécanique.

8. Système selon l'une quelconque des revendications 1 à 3, les instructions amenant le processeur (322) à déterminer si le cycle respiratoire est valide en déterminant qu'aucun artefact n'a affecté le cycle respiratoire.

9. Système selon la revendication 8, l'artefact comprenant une déconnexion de capteur, une modification de la PEP, une purge et une fuite.

10. Procédé de détection d'un événement respiratoire asynchrone, comprenant : la récupération d'une forme d'onde de données d'impédance à partir d'un dispositif de tomographie par impédance électrique ; la récupération d'une ou de plusieurs formes d'onde de débit et de pression ;

l'alignement de la forme d'onde des données d'impédance et l'une ou plusieurs des formes d'onde de débit et de pression par rapport au temps ;
la comparaison de la forme d'onde des données d'impédance et l'une ou plusieurs des formes d'onde de débit et de pression ;
la détermination de la survenue d'un événement respiratoire asynchrone en comparant la forme d'onde des données d'impédance et l'une ou plusieurs des formes d'onde de débit et de pression ;
la classification de l'événement respiratoire asynchrone ; et
la fourniture des informations identifiant l'événement respiratoire asynchrone ;
la classification de l'événement respiratoire asynchrone consistant à déterminer si l'événement respiratoire asynchrone est un événement à double déclenchement.

11. Procédé selon la revendication 10, comprenant en outre l'estimation d'un moment d'un effort musculaire d'un patient.

12. Procédé selon la revendication 11, la classification de l'événement respiratoire asynchrone consistant à déterminer si le moment de l'effort musculaire du patient a déclenché un premier cycle de l'événement respiratoire asynchrone.

**FIG. 1**

**FIG. 2**

FIG. 3

FIG. 4

_500_

| 502 | DETECT MISSING CYCLES |
| 504 | DETECT ARTIFACTS |
| 506 | DETECT ASYNCHRONIES |
| 508 | COMPLETE DETECTION OF ARTIFACTS AND ASYNCHRONIES |

**FIG. 5**

**FIG. 6A**

**FIG. 6B**

700

702 — RECENT PURGE → 704 INVALID CYCLE

706 — RECENT SENSOR DISCONNECTION → 708 INVALID CYCLE

710 — PATIENT UNDER VENTILATORY SUPPORT → 712 INVALID CYCLE

714 — LEAK → 716 INVALID CYCLE

718 — PEEP CHANGE → 720 INVALID CYCLE

722 — VALID CYCLE

**FIG. 7**

**FIG. 8**

**FIG. 9**

Map of
ventilation

Distribution of
ventilation

1002

1012

1004

1006

1014

1008

1010

A

D

E

P

D          E

67%    33%

71%

29%

A

P

**FIG. 10A**

Map of
ventilation

Distribution of
ventilation

1002

1004

1006

1008

1010

A

D

E

P

D          E

56%    44%

46%

54%

A

P

**FIG. 10B**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021074747 A1